# EUROPEAN PATENT APPLICATION

(11) **EP 2 072 495 A1**
(43) Date of publication of application: **24.06.2009**
(21) Application number: 07123985.9
(22) Date of filing: 21.12.2007
(51) Int. Cl.: C07C 213/02, C07C 217/74

(54) **Preparation of phenethyl tertiary amine derivatives**

(71) Applicant: Siegfried Ltd., 4800 Zofingen (CH)
(72) Inventor: Schlegel, Jens, 5034, Suhr (CH); Demel, Peter, 79618, Rheinfelden (DE)
(74) Representative: Sandmann, Wolfgang

(57) **Abstract**

The present invention relates to a process for the preparation of phenethyl tertiary amine derivatives by hydrogenation of phenylacetonitriles.

More particularly, the present invention relates to a one pot two step process for the preparation of phenethyl tertiary amine derivatives.

## Description

The present invention relates to a process for the preparation of phenethyl tertiary amine derivatives by hydrogenation of phenylacetonitriles. More particularly, the present invention relates to a one pot two step process for the preparation of phenethyl tertiary amine derivatives.

Venlafaxine is one a representative for the phenethyl tertiary amine derivatives of the present invention, and the international non-proprietary name (INN) for 1-[2-dimethylamino-1-(4-methoxyphenyl)ethyl]-cyclahexanol of formula (Ia) 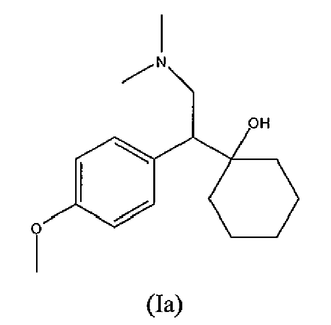 and/or physiologically or pharmaceutically acceptable salts thereof.

Venlafaxine and pharmaceutically acceptable salts thereof, in particular the hydrochloride salt, are known as antidepressants and are widely used. Usually, the racemic mixture is used, i.e. (±)-1-[2-dimethylamino-1-(4-methoxyphenyl)ethyl]-cyclohexanol.

The preparation of Venlafaxine is well known and described, for example, in US 4 535 186. In this document, the final steps towards the preparation of Venlafaxine comprise the reduction of a nitrile to an amine and the subsequent methylation of the amine to form the tertiary amine of Venlafaxine. However, the described process results in low yield and an undesired amount of by-products.

A similar approach is described in EP 1 238 965, wherein the reduction and direct subsequent methylation of 1-[cyano-(4-methoxyphenyl)methyl]cyclohexanol (or (1-hydroxy-cyclohexyl)-(4-methoxy-phenyl)-acetonitrile) of formula (IIa) 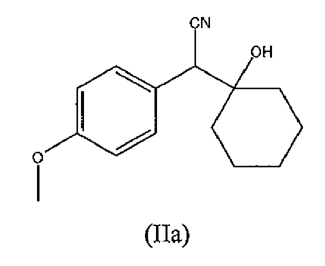 is achieved with the addition of formalin and hydrogen at 1,38 MPa (200 psi) in the presence of Raney nickel as catalyst. This one pot process can only provide yields below 30%.

The object of the present invention is to provide a process for the preparation of Venlafaxine and related compounds in high yield.

A first aspect of the present invention refers to a method for the preparation of a compound of general formula (I) 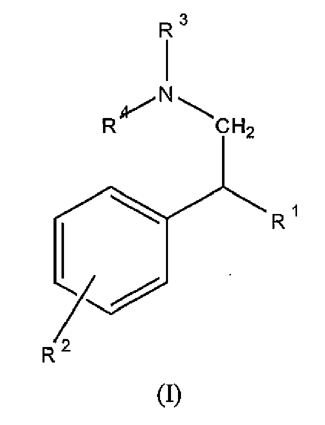 wherein
R¹ represents a linear, branched or cyclic, substituted or unsubstituted alkyl;
R² represents hydrogen, a linear, branched or cyclic, substituted or unsubstituted alkyl or alkoxy; and
R³ and R⁴, independently, represent a linear, branched or cyclic, substituted or unsubstituted alkyl;
comprising the steps of
a. reacting a nitrile of formula (II) 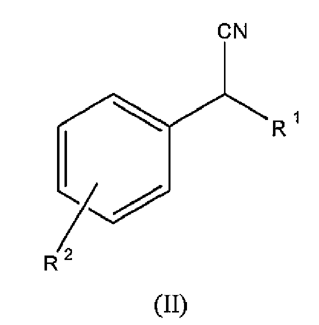 wherein R¹ and R² are defined as above;
   in a solvent in the presence of
   an acid;
   hydrogen (H₂); and
   palladium as a catalyst
   to form a compound of formula (III) as an intermediate compound; 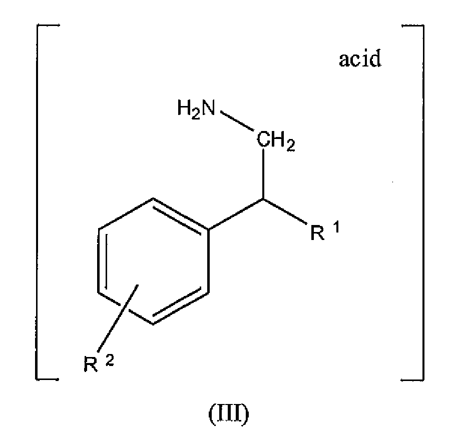 wherein R¹ and R² are defined as above;
b. adding to the reaction mixture of step a.
   a base;
   an aldehyde; and
   hydrogen (H₂),
   and reacting the mixture to achieve the compound of formula (I).

The process of the present invention allows for high yields, a low rate of OH-cleavage, and less by-products, like secondary amines.

The method of the first aspect is a one pot two step reaction wherein the intermediate compound of formula (III) is not isolated, but directly converted to the desired product of formula (I).

As starting material for the reaction, a compound of formula (II) is used. This compound is readily available to a person skilled in the art.

In any of the compounds of formulae (I) - (III), R¹ represents a linear, branched or cyclic, substituted or unsubstituted alkyl, and R² represents hydrogen, a linear, branched or cyclic, substituted or unsubstituted alkyl or alkoxy.

In a preferred embodiment, R¹ represents a linear, branched or cyclic, substituted or unsubstituted alkyl having 1 to 10 carbon atoms, preferably having 1 to 6 carbon atoms, more preferabyl a substituted or unsubstituted cyclohexane, most preferably a 1-hydroxy substituted cyclohexane.

In another preferred embodiment, R² represents hydrogen, a linear, branched or cyclic, substituted or unsubstituted alkyl or alkoxy having 1 to 10 carbon atoms, preferably 1 to 6 carbon atoms, and more preferably, R² is selected from hydrogen, methyl, ethyl, propyl, isopropyl, methoxy, ethoxy, propoxy and isopropoxy, with methoxy being the most preferred.

The substitution of R² can be at any position of the phenyl ring, i.e. in ortho, meta, or para position in relation to the ethylamine. In a preferred embodiment, substituent R² is in para position.

Both steps a. and b. of the reaction are carried out in a solvent. As the reaction is a one pot reaction in two steps, the solvent is the same in both steps of the reaction.

In a preferred embodiment, the solvent is a protic polar solvent, preferably an alcohol, and most preferably selected from methanol, ethanol, isopropanol and mixtures thereof. The most preferred solvent is methanol.

In another preferred embodiment, the solvent is an aprotic polar solvent, preferably selected from dichloromethane, toluene, ethyl acetate and mixtures thereof.

In general, the solvent may be used as a dry solvent, or as commercially available industry standard solvent without any need to further dry the solvent. The presence of small amounts of water will not influence the reaction, as some water may be introduced by the addition of the catalyst.

In still another preferred embodiment, the compound of formula (II) is present in the solvent in a mole fraction in the range of 0.01 - 1, preferably in the range of 0.05 - 0.5, more preferably in the range of 0.1 to 0.4, and most preferably in the range of 0.2 - 0.3.

In yet another preferred embodiment, the compound of formula (II) is present in the solvent in an amount of up to 100 % by weight, based on the amount of solvent, preferably in an amount in the range of 5 to 50 % by weight, more preferably in the range of 10 to 40 % by weight, and most preferably in the range of 20 to 30 % by weight.

The first step a of the conversion comprises the hydrogenation of the nitrile group of compound (II). This hydrogenation is carried out in the presence of a palladium(0) catalyst.

In a preferred embodiment, the palladium catalyst is a carbon supported palladium(0) catalyst, preferably wherein the palladium loading on the carbon support is in the range of 1 - 10% by weight, more preferably 2 - 8% by weight, even more preferably 4 - 6% by weight, and most preferably about 5% by weight, in respect to the carbon support. It is, however, also possible to use other supported palladium catalysts, like palladium supported on barium sulfate.

In another preferred embodiment, the palladium catalyst is used in a weight proportion of more than 0.5 % by weight, preferably more than 1 % by weight, more preferably more than 1.25 % by weight, of palladium, based on the amount by weight of compound of formula (II). The catalyst may be used either as dry catalyst, or as a humid catalyst still carrying some amount of water. Small amounts of wafer will not disturb the reaction.

The hydrogenation reaction is carried out in the presence of an acid, Preferably, the reaction is carried out in an organic or inorganic acid, preferably in a strong organic or inorganic acid. The use of a strong organic or inorganic acid is preferred over the use of a weak acid as the conversion, the yield and the purity will decrease with the use of a weak acid.

Examples of weak acids are organic dicarboxylic acids, like oxalic acid or citric acid; or inorganic acids, like phosphoric acid or hydrochloric acid.

As strong acids, preferably a sulfonic acid, like methanesulfonic acid, ethanesulfonic acid, benzenesulfonic acid, or toluenesulfonic acid, more preferably p-toluenesulfonic acid; sulfuric acid; perchloric acid; an organic carboxylic acid, like formic acid or acetic acid, are used. In a preferred embodiment, the acid is selected from methanesulfonic acid, ethane sulfonic acid, benzenesulfonic acid and toluenesulfonic acid, preferably p-toluenesulfonic acid.

In still another embodiment, the acid is present in the reactions mixture of step a. with at least 0.1 eq, preferably at 0.1 - 2.0 eq, more preferably at 0.8 - 1,5 eq, even more preferably at 0.9 - 1.2 eq, and most preferably at about 1 eq, in respect to the compound of formula (II), wherein eq refers to the molar equivalent. In another preferred embodiment, the acid is present in the reaction mixture of step a. with at least 0.9 eq, more preferably at least 1 eq, in respect to the compound of formula (II).

The use of an acid is a central element of the present invention. With the use of an acid, the yield and the conversion of the reaction are increased. Without being bound to theory, the inventors believe that the nitrile function is activated by the acid. Additionally, the resulting amine precipitates and the amount of side products is decreased. As a consequence, the product has a higher purity.

In a first substep of step a., the compound of formula (II), the catalyst and the acid are suspended and/or dissolved in a solvent. Preferably, the acid is dissolved in the solvent prior to the addition of the compound of formula (II) and the catalyst, as the dissolving of the acid usually is an exothermic reaction.

As both steps are hydrogenation reactions, the reactions are carried out in an autoclave constructed for hydrogenation reactions at higher pressure and equipped with a stirring means, like e.g. a mechanical stirring means.

The hydrogenation of the nitrile function of compound (II) is carried out in the presence of hydrogen (H₂). In a preferred embodiment of step a., the hydrogen is applied at a pressure in the range of 1 -100 bar, preferably in the range of 2 - 60 bar, more preferably in the range of 5 - 40 bar, and most preferred in a range of 5 - 15 bar.

While the hydrogenation reaction is carried out, the reaction mixture is stirred, and the temperature is preferably maintained in step a. in the range of 0 - 40°C, preferably in the range of 5 - 30°C, more preferably in the range of 10 - 25°C, and most preferably in the range of 15 - 22°C.

If the temperature is too high, the amount of undesired side products or by-products is increased, and if the temperature is too low, the conversion is very slow. The temperature is thus preferably maintained within the above given ranges.

The reaction temperature and the hydrogen pressure are maintained for a period of time to ensure the complete or substantially complete conversion of the compound of formula (II) to the compound of formula (III). In a preferred embodiment, the reaction of step a. is carried out over a period of time in the range of 1 to 10 hours, preferably in the range of 2.5 to 4 hours.

After the reaction of step a. is finished, the resulting reaction mixture is converted in a subsequent step b. This step can follow immediately, or the reaction mixture is stored, preferably at lower temperatures, until step b. is carried out.

In the resulting compound of formula (I) after carrying out the reaction of step b., the residues R³ and R⁴, independently, represent a linear, branched or cyclic, substituted or unsubstituted alkyl. In a preferred embodiment, R³ and R⁴, independently, represent a linear, branched or cyclic, substituted or unsubstituted alkyl having 1 to 6 carbon atoms, preferably having 1 to 4 carbon atoms, more preferably a methyl, ethyl or propyl, with methyl as R³ and R⁴ being most preferred.

After the hydrogenation of the nitrile group is completed, a base and an aldehyde are added to the reaction mixture resulting from step a.

In a preferred embodiment, the base is an inorganic base, preferably lithium hydroxide, potassium hydroxide, and most preferably sodium hydroxide; or an organic base, preferably triethyl amine. The base may be added as an aqueous solution, e.g. a 30% sodium hydroxide aqueous solution.

In another preferred embodiment, the amount of base added to the reaction mixture is depending from the amount of acid added to the reaction mixture in step a. and the amount of starting material of formula (II). Preferably, the molar amount of base added exceeds the amount of acid added and not used by the amount of amine of formula (III) produced in step a. In other words, the molar amount of base exceeds the difference of the molar amount of acid and the molar amount of starting material of formula (II), i.e. n(acid) - n(compound II) < n(base), wherein n is the amount of substance (in mole). If the amount of base is not sufficient to produce some amount of free base of the intermediate product of formula (III), the reaction of step b. will not work properly. The maximum amount of base added is not restricted. For economical and environmental reasons, however, the molar amount of base should not exceed the molar amount of acid added in step a. Preferably, the amount of base added results in an amount of free base of the compound of formula (III) of at least 0.001 eq, in respect to the total molar amount of compound of formula (II). More preferably, the amount results in an amount of at least 0.01 eq, even more preferably 0.1 eq, of free base of the compound of formula (III). However, it is also possible to add more base to result in an amount of free base equivalent to the amount of compound of formula (III), i.e. all of the compound of formula (III) is present as free base. This can be achieved by adding the same amount of base, or even more, e.g. twice the amount of base, as the amount of acid added in step a.

The aldehyde may be any aldehyde, and will be selected by a person skilled in the art depending from the residues R³ and R⁴ to be introduced. In a preferred embodiment, the aldehyde is an aldehyde of the general formula R^{a}C(O)H with R^{a} being selected from hydrogen, alkyl, cycloalkyl and aryl, preferably wherein R^{a} is hydrogen, C₁-₅ alkyl, more preferably methyl or ethyl. In a particularly preferred embodiment, the aldehyde is formaldehyde or paraformaldehyde (polyoxymethylene). The aldehyde may be added in solution, especially as aqueous solution, or as it is. In another preferred embodiment, the aldehyde is formaldehyde added as a solution of 35 % in water.

In a preferred embodiment, the aldehyde is added in an amount of 1.9 - 4 eq, preferably in 1.95 - 3 eq, and most preferably 2 - 2.5 eq, in respect to the amount of compound of formula (II).

The second step b. of the reaction for the preparation of the compound of formula (II) comprises another hydrogenation reaction. The reaction is thus carried out in the presence of hydrogen (H₂). In a preferred embodiment of step b., the hydrogen is applied at a pressure in the range of 1 -100 bar, preferably in the range of 2 - 60 bar, more preferably in the range of 5 - 40 bar, and most preferred in a range of 5 - 15 bar.

During the hydrogenation in step b., the reaction mixture is preferably heated to a temperature in the range of 20 - 150°C, preferably in the range of 40 - 120°C, more preferably in the range of 40 - 100°C, even more preferably in the range of 60 - 95°C, and most preferably in the range of 80 - 95°C.

The reaction temperature and the hydrogen pressure in step b. are maintained for a period of time to ensure the complete or substantially complete conversion of the intermediate compound of formula (III) to the compound of formula (I). In a preferred embodiment, the reaction of step b. is carried out over a period of time in the range of 1 to 10 hours, preferably in the range of 2.5 to 4 hours.

Each of the residues R¹, R², R³ and R⁴ is, individually, selected from the same groups for each of the compounds of formulae (I)-(III). In an especially preferred embodiment of the invention, the residues are selected to form Venlafaxine of formula (Ia).

Another aspect of the present invention refers to the preparation of the hydrochloric salt of the compound of formula (I). The preparation comprises
a. suspending and/or dissolving the compound of formula (I) in a solvent;
b. heating the suspension prepared in step a. to the reaction temperature;
c. adding hydrogen chloride gas to the suspension prior to, while or after heating;
d. distillation of the solvent;
e. subsequent to step d., recovering the hydrochloric salt of the compound of formula (I) by filtration.

In a first step of the preparation of the hydrochloric salt, the compound of formula (I) is suspended and/or dissolved in a solvent or solvent mixture. In the following, the terms suspension and solution will be used equivalently and interchangeably. Even if the compound of formula (I) is completely dissolved in the solvent or solvent mixture, the resulting solution may be termed as suspension, or vice versa, the suspension of compound of formula (I) may be termed as solution.

In a preferred embodiment, the solvent is a mixture of ethyl acetate and an alcohol. More preferably, the alcohol is selected from methanol, ethanol, n-propanol and iso-propanol, with ethanol being preferred. Further preferred, the weight ratio of ethyl acetate : alcohol is in the range of 0.1 : 10 to 10 : 0.1, preferably in the range of 0.5 : 2 to 2 : 0.5, more preferably in the range of 0.9 : 1.1 to 1.1 : 0.9, and most preferably about 1, i.e. about equal amounts of ethyl acetate and alcohol are used. The ratio of the two solvents is referred to as weight ratio.

In another preferred embodiment, the solvent is an alcohol, preferably selected form methanol, ethanol, n-propanol and iso-propanol, with ethanol being preferred. In this embodiment, i.e. if only an alcohol is used as solvent, ethyl acetate is added prior to the distillation in step d.

In general, the use of ethanol as alcohol is preferred over other alcohols as a transesterification of ethyl acetate will not result in different acetates.

As solvent or solvent mixture, dry solvents can be used. However, it is not necessary to use dry solvents. Commercially available solvents of industrial standard can also be used. Small amounts of water are acceptable for the formation of the Venlafaxine salt and will be removed during the removal of the alcohol.

The next two steps of the method of preparation are the heating of the suspension and the addition of hydrogen chloride gas. These steps are interchangeable, and can even be carried out simultaneously. As the reaction of the hydrogen chloride gas with the basic compound of formula (I) is exothermic, this reaction can even be used to heat the reaction mixture. Thus, the hydrogen chloride gas can be added prior to the heating, while heating, or even after having reached the reaction temperature. The addition of the hydrogen chloride gas can be achieved e.g. by bubbling the gas through the reaction mixture.

After the reaction mixture has reached the reaction temperature, the temperature is maintained for at least 0.25 to 6 hours, preferably for 0.5 to 4 hours, and more preferably for 1 to 2 hours.

If the reaction mixture is maintained at the reaction temperature for a too short period, it will be more difficult to filter the resulting crystals from the solvent.

In a preferred embodiment, the reaction temperature is the reflux temperature of the solvent or solvent mixture. In another preferred embodiment, the reaction temperature is in the range of room temperature to 150°C, more preferably in the range of 30°C to 100°C, and even more preferably in the range of 70-90°C. Most preferably, the temperature is in the range of 75-80°C.

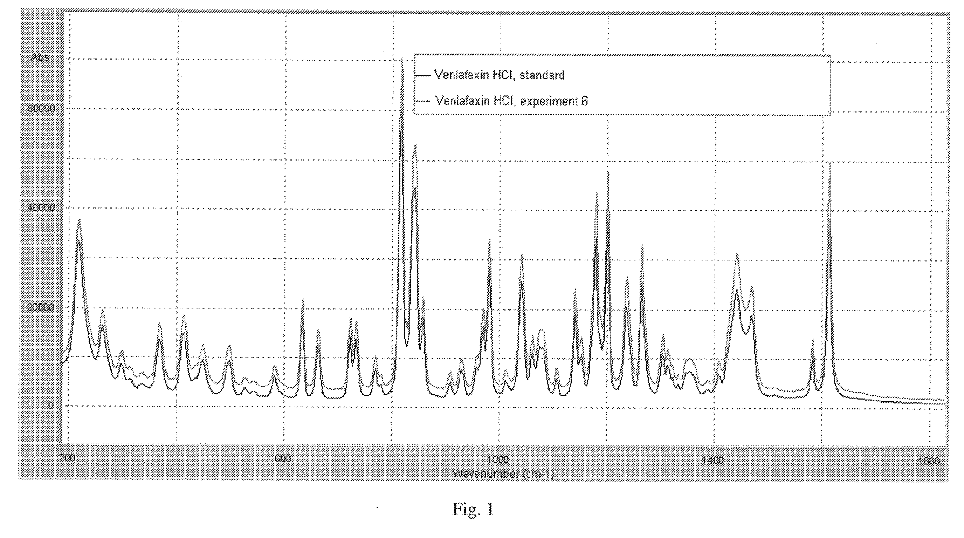

The temperature as well as the period of time, the reaction mixture is maintained at this temperature, is relevant for the formation of the polymorph as well as the particle size. A Raman spectrum of a preferred polymorph which can be achieved, amongst others, with the process of the present invention is shown in Fig. 1. It should, however, be noted that other polymorphs can also be prepared by the process of the present invention.

After having reached the reaction temperature and while the reaction mixture is maintained at this temperature, the pH of the reaction mixture may be adjusted. In a preferred embodiment, hydrogen chloride gas is added until the reaction mixture has reached a pH in the range of 1-4, preferably in the range of 2-3, and more preferably about 2.5. If the pH of the reaction mixture is above the aimed value, more hydrogen chloride gas is added. On the other hand, the pH of the reaction mixture can be lowered by further addition of the basic compound of formula (I).

The pH of the reaction mixture can be measured while the reaction mixture is at the reaction temperature. If the pH is too low, i.e. the amount of hydrogen chloride added is too high and the reaction mixture is too acidic, the final hydrogen chloride salt will be in the form of a very fine powder, and it will be more difficult to recover the salt. On the other hand, if the pH is too high, i.e. the reaction mixture is too basic, the yield of the hydrochloric salt will be reduced. The best pH for the preparation of the salt is depending from the pKₛ of the compound of formula (I), and can be determined by a person skilled in the art by routine experimentation.

After the reaction mixture has reached the target pH, i.e. after the addition of the hydrogen chloride gas, and the reaction mixture was maintained at this pH for a period of time in order to achieve the conversion, the alcohol is removed from the reaction mixture (step d.).

In order to yield a very clean product in high yield, the alcohol is removed and/or replaced by ethyl acetate. If only an alcohol is used as a solvent during the preparation of the hydrochloride, ethyl acetate is added prior to the distillation of the alcohol. In a preferred embodiment, the resulting solvent mixture has a weight ratio of ethyl acetate : alcohol in the range of 0.1 : 10 to 10 : 0.1, preferably in the range of 0.5 : 2 to 2 : 0.5, more preferably in the range of 0.9 : 1.1 to 1.1 : 0.9, and most preferably about 1, i.e. about equal amounts of ethyl acetate and alcohol are used. The ratio of the two solvents is referred to as weight ratio.

When removing the alcohol from the solvent mixture, the alcohol is preferably distilled off as an azeotrope.

In a preferred embodiment, the removal of the alcohol, preferably ethanol, is achieved in several steps comprising removal of the alcohol as an azeotrope and replacing the removed amount of solvent mixture by ethyl acetate. If the removal of the alcohol is performed batchwise, in an even more preferred embodiment, the amount, by weight, of solvent removed in the first step corresponds to the amount, by weight, of alcohol added to the reaction mixture in step a. As the solvent removed as azeotrope also contains some ethyl acetate, the azeotropic distillation is continued with the addition of further ethyl acetate. The addition and removal of solvent can be performed batchwise. In this case, the amount of solvent removed is replaced by approximately the same amount of ethyl acetate, which amount, by weight, is then in turn removed by azeotropic distillation. The amount of distillate removed as an azeotrope is within a range of 80 - 120 % by weight, preferably 80 - 110 % by weight, of the amount of ethyl acetate added. This process of addition of ethyl acetate and azeotropic removal can be repeated several times. In a still preferred embodiment, the addition and removal is repeated once, more preferred twice, even more preferred three times, still more preferred four times, or even more often.

In another preferred embodiment, the removal of the alcohol is performed in a continuous process. While the alcohol is removed as an azeotrope, additional ethyl acetate is added to the reaction mixture. Preferably, the amount of ethyl acetate replaces the amount of solvent mixture removed as azeotrope, based on the amount by weight. Preferably, the amount of azeotrope removed by distillation is continuously replaced by an amount of ethyl acetate in a range of 80 -120% by weight, based on the amount of azeotrope removed, more preferably in the range of 80 -110 % by weight.

In still another preferred embodiment, the amount of solvent removed from the reaction mixture does not exceed 50 % by weight, based on the total amount of solvent at the beginning of the distillation, preferably it does not exceed 40 % by weight, more preferably 25 % by weight.

After the complete or substantially complete removal of the alcohol from the reaction mixture by azeotropic distillation, the reaction mixture is slowly cooled. In a preferred embodiment, the reaction mixture is cooled to a target temperature in the range of 10-20°C, preferably to about 15°C. In another preferred embodiment, the cooling is performed at a cooling rate not exceeding 1°C/min.

After the reaction mixture is cooled to a target temperature, the suspension is stirred at this temperature for a period of at least 1 hour, preferably at least 5 hours, more preferably at least 10 hours, and most preferably at least 15 hours, prior to recovering the salt.

The final product is then recovered by filtration of the salt from the reaction mixture. In still another embodiment, the salt is washed with a solvent, preferably with a solvent mixture of ethyl acetate and alcohol, further preferred at a weight ratio of ethyl acetate : alcohol of 98 : 2. The alcohol is preferably selected from methanol, ethanol, n-propanol and iso-propanol, with ethanol being preferred. The final product may then be dried at reduced pressure and/or elevated temperature.

The method described above allows for the preparation of very pure venlafaxine hydrochloride at high yields. Further, the crystal form of the resulting venlafaxine hydrochloride can be reproduced and the particle size of the crystals is very uniform. A further advantage of the method is the possibility of recycling of the solvent(s), as the solvents are still pure and not polluted by any by-products or venlafaxine.

The following experiments are given demonstrative purposes only, without limiting the scope of the invention.

### Experiments

### Experiment 1 Preparation of venlafaxine

A 5 L reactor is charged with 1.4 kg of methanol and 218 g (2.27 mol) of methane sulfonic acid is added at 0-30 °C. 543 g (2.21 mol) 1-[cyano-(4-methoxyphenyl)methyl]cyclohexanol and 27.2 g (5% w/w) 5% Palladium on charcoal (50% water wet) are added and the mixture is hydrogenated at 15 - 25 °C with a pressure of 9-10 bar for 4 h. 15 g (0.11 mol) 30% aqueous sodium hydroxide solution and 407 g (4.88 mol) 36% aqueous formaldehyde solution are added to the suspension at 20 °C and the mixture is hydrogenated at a pressure of 9-10 bar and the temperature is increased during hydrogenation from 20 to 90 °C and stirred for a total of 4 h. The palladium catalyst is filtered at 20°C and washed with 200 g of methanol and 200 g of water. The combined layers are concentrated in vacuum and the residual oil is dissolved in 1.0 kg of ethyl acetate and the organic phase is washed with a mixture of 500 g of potassium carbonate in 1.0 kg of water and finally with 5% sodium chloride solution. The organic solvent is removed in vacuum and the remaining oil is crystallized from a solution of 460 g acetone and 280 g water. The colourless crystals are filtered and washed with additional solution of 134 g acetone and 66 g of water. The crystals are dried in vacuum, p = 10 - 100 mbar, at 40 - 60 °C to yield 461 g (75 %) of venlafaxine.

### Experiment 2 Preparation of venlafaxine

A 5 L reactor is charged with 1.4 kg of methanol and 218 g (2.27 mol) of methane sulfonic acid is added at 0-30 °C. 543 g (2.21 mol) 1-[cyano-(4-methoxyphenyl)methyl]cyclohexanol and 27.2 g (5% w/w) 5% Palladium on charcoal (50% water wet) are added and the mixture is hydrogenated at 15 - 25 °C with a pressure of 4-5 bar for 4 h. 15 g (0.11 mol) 30% aqueous sodium hydroxide solution and 407 g (4.88 mol) 36% aqueous formaldehyde solution are added to the suspension at 20 °C and the mixture is hydrogenated at a pressure of 4-5 bar and the temperature is increased during hydrogenation from 20 to 90 °C and stirred for a total of 4 h. The palladium catalyst is filtered at 20 °C and washed with 200 g of methanol and 200 g of water. The combined layers are concentrated in vacuum and the residual oil is dissolved in 1.0 kg of ethyl acetate and the organic phase is washed with a mixture of 500 g of potassium carbonate in 1.0 kg of water and finally with 5% sodium chloride solution. The organic solvent is removed in vacuum and the remaining oil is crystallized from a solution of 460 g acetone and 280 g water. The colourless crystals are filtered and washed with additional solution of 134 g acetone and 66 g of water. The crystals are dried in vacuum, p = 10 - 100 mbar, at 40 - 60 °C to yield 433 g (71 %) of venlafaxine.

### Experiment 3 Preparation of venlafaxine

A 2 L reactor is charged with 512 g of methanol and 70.3 g of methane sulfonic acid is added at 0-30 °C. 173.9 g 1-[cyano-(4-methoxyphenyl)methyl]cyclohexanol and 8.7 g (5% w/w) 5% Palladium on charcoal (50% water wet) are added and the mixture is hydrogenated at 15 - 25 °C with a pressure of 40 bar for 3 h. 4.8 g 30% aqueous sodium hydroxide solution and 130.8 g 36% aqueous formaldehyde solution are added to the suspension at 20 °C and the mixture is hydrogenated at a pressure of 40 bar and the temperature is increased during hydrogenation from 20 to 90°C and stirred for a total of 75 min. The palladium catalyst is filtered at 20 °C and washed with 10 g of methanol and 10 g of water. The combined layers are concentrated in vacuum and the residual oil is dissolved in 322 g of ethyl acetate and the organic phase is washed with a mixture of 96 g of potassium carbonate in 260 g of water and finally with 5% sodium chloride solution. The organic solvent is removed in vacuum and the remaining oil is crystallized from a solution of 148 g acetone and 91 g water. The colourless crystals are filtered and washed with additional solution of 44 g acetone and 22 g of water. The crystals are dried in vacuum, p = 10 --100 mbar, at 40 - 60 °C to yield 106 g (74 %) of venlafaxine.

### Experiment 4 Preparation of venlafaxine

A 2 L reactor is charged with 512 g of methanol and 70.3 g of methane sulfonic acid is added at 0-30 °C. 173.9 g 1-[cyano-(4-methoxyphenyl)methyl]cyclohexanol and 6.9 g (4% w/w) 5% Palladium on charcoal (50% water wet) are added and the mixture is hydrogenated at 15 - 25 °C with a pressure of 40 bar for 3.5 h. 4.8 g 30% aqueous sodium hydroxide solution and 130.8 g 36% aqueous formaldehyde solution are added to the suspension at 20 °C and the mixture is hydrogenated at a pressure of 40 bar and the temperature is increased during hydrogenation from 20 to 90 °C and stirred for a total of 60 min. The palladium catalyst is filtered at 20 °C and washed with 10 g of methanol and 10 g of water. The combined layers are concentrated in vacuum and the residual oil is dissolved in 322 g of ethyl acetate and the organic phase is washed with a mixture of 96 g of potassium carbonate in 260 g of water and finally with 5% sodium chloride solution. The organic solvent is removed in vacuum and the remaining oil is crystallized from a solution of 148 g acetone and 91 g water. The colourless crystals are filtered and washed with additional solution of 44 g acetone and 22 g of water. The crystals are dried in vacuum, p = 10 -- 100 mbar, at 40 - 60 °C to yield 104 g (74 %) of venlafaxine.

### Experiment 5 Preparation of venlafaxine

A 2 L reactor is charged with 513 g of methanol and 70.3 g of methane sulfonic acid is added at 0-30 °C. 173.7 g 1-[cyano-(4-methoxyphenyl)methyl]cyclohexanol and 5.2 g (3% w/w) 5% Palladium on charcoal (50% water wet) are added and the mixture is hydrogenated at 15 - 25 °C with a pressure of 40 bar for 4.5 h. 4.8 g 30% aqueous sodium hydroxide solution and 130.8 g 36% aqueous formaldehyde solution are added to the suspension at 20 °C and the mixture is hydrogenated at a pressure of 40 bar and the temperature is increased during hydrogenation from 20 to 90 °C and stirred for a total of 120 min. The palladium catalyst is filtered at 20 °C and washed with 10 g of methanol and 10 g of water. The combined layers are concentrated in vacuum and the residual oil is dissolved in 322 g of ethyl acetate and the organic phase is washed with a mixture of 96 g of potassium carbonate in 260 g of water and finally with 5% sodium chloride solution. The organic solvent is removed in vacuum and the remaining oil is crystallized from a solution of 148 g acetone and 91 g water. The colourless crystals are filtered and washed with additional solution of 44 g acetone and 22 g of water. The crystals are dried in vacuum, p = 10 -- 100 mbar, at 40 - 60 °C to yield 95.6 g (67 %) of venlafaxine,

### Experiment 6 Preparation of venlafaxine hydrochloride

In a 1-L-reactor, 64 g (0.231 mol) of venlafaxine are added to a mixture of 170 g of ethyl acetate and 170 g of ethanol. After the reaction mixture is heated to the reaction temperature (approx. 68-73°C), 8 g of hydrogen chloride gas is bubbled through the reaction mixture. The reaction mixture is maintained at the reaction temperature for 15 minutes, and then the pH is determined to be 2.5. At a temperature of 64°C, 170 g of solvent are removed as azeotrope. During the removal of the solvent, a precipitate is formed. After the removed solvent is replaced by 170 g of ethyl acetate, again 170 g of solvent are removed as azeotrope. The process of addition and removal of solvents is repeated two more times. Then, the reaction mixture is cooled to 15°C at a cooling rate of 1°C/min, and the reaction mixture is maintained at this temperature for 15 hours. The product is filtered by suction filtration and washed twice with 80 g of a mixture of ethyl acetate and ethanol (98:2 w/w). The white crystals are dried at 100 mbar and 50°C for 15 hours to yield 69 g of venlafaxine HCl (yield: 95%) at a purity of more than 99.8%. The raman spectrum of the resulting polymorph is given in Fig. 1.

## Claims

1. Method for the preparation of a compound of general formula (I) 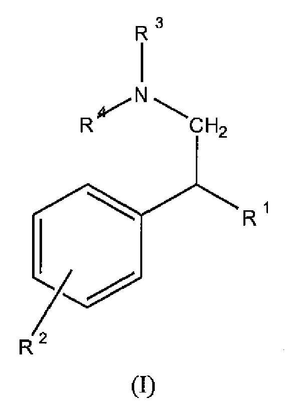 wherein
R¹ represents a linear, branched or cyclic, substituted or unsubstituted alkyl;
R² represents hydrogen, a linear, branched or cyclic, substituted or unsubstituted alkyl or alkoxy; and
R³ and R⁴, independently, represent a linear, branched or cyclic, substituted or unsubstituted alkyl;
comprising the steps of
a. reacting a nitrile of formula (II) 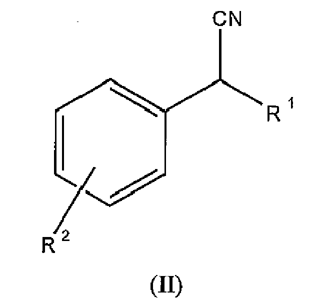 wherein R¹ and R² are defined as above;
in a solvent in the presence of
an acid;
hydrogen (H₂); and
palladium as a catalyst
to form a compound of formula (III) as an intermediate compound; 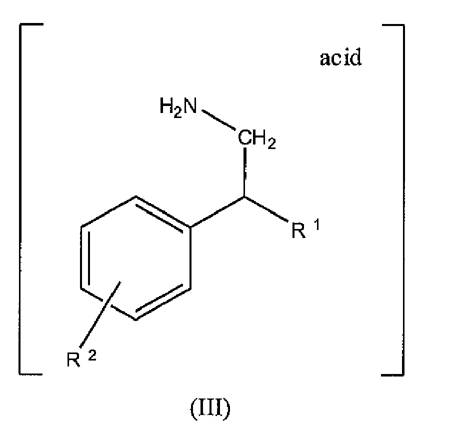 wherein R¹ and R² are defined as above;
b. adding to the reaction mixture of step a.
a base;
an aldehyde; and
hydrogen (H₂),
and reacting the mixture to achieve the compound of formula (I).

2. The method according to claim 1, wherein R¹ represents a linear, branched or cyclic, substituted or unsubstituted alkyl having 1 to 10 carbon atoms, preferably a substituted or unsubstituted cyclohexane, most preferably a 1-hydroxy substituted cyclohexane.

3. The method according to any of the preceding claims, wherein R² represents hydrogen, a linear, branched or cyclic, substituted or unsubstituted alkyl or alkoxy having 1 to 10 carbon atoms, preferably a methoxy or ethoxy.

4. The method according to any of the preceding claims, wherein R² is in the para position of the phenyl.

5. The method according to any of the preceding claims, wherein R³ and R⁴, independently, represent a linear, branched or cyclic, substituted or unsubstituted alkyl having 1 to 6 carbon atoms, preferably a methyl, ethyl or propyl.

6. The method according to any of the preceding claims, wherein the solvent is a protic polar solvent, preferably an alcohol, and most preferably selected from methanol, ethanol, isopropanol and mixtures thereof.

7. The method according to any of the preceding claims, wherein the solvent is an aprotic polar solvent, preferably selected from dichloromethane, toluene, ethyl acetate and mixtures thereof.

8. The method according to any of the preceding claims, wherein the compound of formula (II) is present in the solvent in an amount of up to 100 % by weight, based on the amount of solvent, preferably in an amount in the range of 5 to 50 % by weight, more preferably in the range of 10 to 40 % by weight, and most preferably in the range of 20 to 30 % by weight.

9. The method according to any of the preceding claims, wherein the acid is a sulfonic acid, preferably methanesulfonic acid, ethanesulfonic acid, or toluenesulfonic acid (p-toluenesulfonic acid?); sulfuric acid; perchloric acid; an organic carboxylic acid, preferably formic acid or acetic acid; an organic dicarboxylic acid, preferably oxalic acid or citric acid; or an inorganic acid, preferably phosphoric acid or hydrochloric acid.

10. The method according to any of the preceding claims, wherein the acid is present in the reactions solution with at least 0.1 eq, preferably at 0.1 - 2.0 eq, more preferably at 0.8 - 1.5 eq, even more preferably at 0.9 - 1.2 eq, and most preferably at about 1 eq, in respect to the compound of formula (II).

11. The method according to any of the preceding claims, wherein the palladium catalyst is a carbon supported palladium catalyst, preferably wherein the palladium loading on the carbon support is in the range of 1 - 10% by weight, more preferably 2 - 8% by weight, even more preferably 4 - 6% by weight, and most preferably about 5% by weight, in respect to the carbon support.

12. The method according to any of the preceding claims, wherein the hydrogenation reaction in step a. is carried out at a temperature in the range of 0 - 40°C, preferably in the range of 5 - 30°C, more preferably in the range of 10 - 25°C, and most preferably in the range of 15 - 22°C.

13. The method according to any of the preceding claims, wherein the hydrogen is applied at a pressure in the range of 1 - 100 bar, preferably in the range of 2 - 60 bar, more preferably in the range of 5 - 40 bar, and most preferred in a range of 5 - 15 bar.

14. The method according to any of the preceding claims, wherein the hydrogenation reaction of step a. is carried out over a period of time in the range of 1 to 10 hours, preferably in the range of 2.5 to 4 hours.

15. The method according to any of the preceding claims, wherein in step a. the acid is dissolved in the solvent in a first step, before the compound of formula (II) is added.

16. The method according to any of the preceding claims, wherein the base is an inorganic base, preferably lithium hydroxide, postassium hydroxide, or sodium hydroxide; or an organic base, preferably triethyl amine.

17. The method according to any of the preceding claims, wherein the base is added in an amount to result in an amount of at least 0.001 eq, preferably 0.01 eq of free base of the compound of formula (II).

18. The method according to any of the preceding claims, wherein the aldehyde is an aldehyde of the general formula R^{a}C(O)H with R^{a} being selected from hydrogen, alkyl, cycloalkyl and aryl, preferably wherein R^{a} is hydrogen.

19. The method according to claim 16, wherein R^{a} is hydrogen or a C₁-₅ alkyl, preferably methyl or ethyl.

20. The method according to any of the preceding claims, wherein the aldehyde is added in an amount of 1.9 - 4 eq, preferably in 1.95 - 3 eq, and most preferably 2 - 2.5 eq, in respect to the compound of formula (II).

21. The method according to any of the preceding claims, wherein the hydrogenation reaction of step b, is carried out over a period of time in the range of 1 to 10 hours, preferably in the range of 2.5 to 4 hours.

22. The method according to any of the preceding claims, wherein step b. is carried out at a temperature in the range of 20 -150°C, preferably in the range of 40 - 120°C, more preferably in the range of 40 - 100°C, even more preferably in the range of 60 - 95°C, and most preferably in the range of 80 - 95°C.

23. Method for the preparation of the hydrochloric salt of the compound of formula (I) comprising
a. suspending and/or dissolving the compound of formula (I) in a solvent;
b. heating the suspension of step a. to the reaction temperature;
c. adding hydrogen chloride gas to the suspension of step a. prior to, while or after heating;
d. distillation of the solvent;
e. recovering the hydrochloric salt of the compound of formula (I) by filtration.

24. Method according to claim 23, wherein after step d, additional ethyl acetate is added to the suspension and the distillation of the solvent mixture as an azeotrope is repeated.

25. Method according to claim 24, where the addition and the distillation are repeated carried out at least one, preferably twice, more preferably three times, and most preferably even more often,

26. Method according to any of the claims 23-25, wherein the addition of hydrogen chloride gas is performed until the reaction mixture has reached a pH in the range of 1-4, preferably in the range of 2-3, and more preferably about 2.5.

27. Method according to any of the claims 23-26, wherein the weight ratio of ethyl acetate : ethanol is in the range of 0.1 : 10 to 10 : 0.1, preferably in the range of 0.5 : 2 to 2 : 0.5, more preferably in the range of 0.9 : 1.1 to 1.1 : 0.9, and most preferably about 1.

28. Method according to any of the claims 23-27, wherein the reaction mixture is cooled to a target temperature in the range of 10-20°C, preferably to about 15°C at a cooling rate not exceeding 1°C/min, and the suspension is stirred at the target temperature for a period of at least 1 hour, preferably at least 5 hours, more preferably at least 10 hours, and most preferably at least 15 hours, prior to recovering the salt.
